# EUROPÄISCHE PATENTSCHRIFT

(11) **EP 1 183 047 B1**
(45) Veröffentlichungstag und Bekanntmachung des Hinweises auf die Patenterteilung: **27.08.2003**
(21) Anmeldenummer: 00943785.6
(22) Anmeldetag: 08.06.2000
(51) Int. Cl.: A61K 45/08, A61K 38/00, A61K 39/00, A61K 35/00, A61K 39/395, A61K 47/48, A61P 35/00

(54) **PROTEINENTHALTENDE PHARMAZEUTISCHE ZUBEREITUNG**
PHARMACEUTICAL PREPARATION
PREPARATION PHARMACEUTIQUE

(30) Priorität: 12.06.1999 DE 19926877; 17.05.2000 DE 10024301
(43) Veröffentlichungstag der Anmeldung: 06.03.2002
(73) Patentinhaber: bitop Aktiengesellschaft für biotechnische Optimierung, 58453 Witten (DE)
(72) Erfinder: BARTH, Stefan, D-50931 Köln (DE)
(74) Vertreter: Meyers, Hans-Wilhelm, Dr.Dipl.-Chem.
(86) Internationale Anmeldenummer: EP0005309
(87) Internationale Veröffentlichungsnummer: WO00076528

(56) Entgegenhaltungen:
- WO-A-95/01154
- WO-A-97/04801
- WO-A-97/38685
- DE-A- 4 244 580
- DE-A- 19 933 466
- US-A- 5 569 457
- US-A- 5 869 050
- AUDRAN REGINE ET AL: "Enhanced immunogenicity of microencapsulated tetanus toxoid with stabilizing agents." PHARMACEUTICAL RESEARCH (NEW YORK), Bd. 15, Nr. 7, Juli 1998 (1998-07), Seiten 1111-1116, XP000990406 ISSN: 0724-8741
- SAUER T ET AL: "ECTOINE - BIOTECHNISCHE PRODUKTION UND MOEGLICHE ANWENDUNGSBEREICHE" GIT FACHZEITSCHRIFT FUER DAS LABORATORIUM,DARMSTADT,DE, Bd. 39, Nr. 10, 1995, Seiten 892-896, XP000918716 ISSN: 0016-3538
- DA COSTA ET AL: "An overview of the role and diversity of compatible solutes in bacteria and archaea" ADVANCES IN BIOCHEMICAL ENGINEERING, BIOTECHNOLOGY,SPRINGER, BERLIN,DE, Bd. 61, 1998, Seiten 117-153, XP002099622 ISSN: 0724-6145

## Beschreibung

Die vorliegende Erfindung betrifft eine pharmazeutische Zubereitung und deren Verwendung.

EP-A-0 553 884 beschreibt gereinigte Tetrahydropyridin-Derivate und pharmazeutische Zusammensetzungen enthaltend diese Derivate. Sie eignen sich zum Schutz der DNA vor Schäden durch DNA-bindende Wirkstoffe, chemische Karzinogene und Mutagene und Strahlungsschäden.

Überraschenderweise wurde nun gefunden, dass kompatible Solute sich zur Effektivitätssteigerung von proteinenthaltenden Substanzen eignen. Gegenstand der Erfindung ist daher eine pharmazeutische Zubereitung enthaltend mindestens eine proteinenthaltende Substanz und Ectoin, bevorzugt Hydroxyectoin (L-Ectoin, S,S-ß-Hydroxyectoin).

Die proteinenthaltende Substanz ist ein Antikörper, ein Antikörperfragment, ein rekombinanter mono- oder höhervalenter Antikörper, ein rekombinantes mono- oder hohervalentes Antikörperfragment, ein bispezifischer Antikörper, ein Diabody oder ein davon abgeleitetes Immuntoxin.

Die erfindungsgemäße pharmazeutische Zubereitung eignet sich insbesondere zur Behandlung von Tumorerkrankungen, Autoimmunerkrankungen, chronischen Entzündungen, Allergien, bakteriellen und viralen Infektionen. Außerdem reduziert sie im Laufe der Behandlung auftretende zytotoxische Aktivitäten, insbesondere das "Vascular Leak Syndrome" (VLS). Bei letzterem führt eine direkte Endothelzellschädigung zu einem Verlust von Albumin aus dem Intrazellularraum in den Extrazellularraum; daraus resultiert eine zunehmende Ansammlung interstitieller Flüssigkeit und folglich eine Gewichtszunahme mit Ausbildung vor allem von Ödemen, Hypotonien und Tachykardien.

Die Wirksamkeit der erfindungsgemäßen pharmazeutischen Zubereitung wird durch das folgende Beispiel näher erläutert:

*Einsatz einer pharmazeutischen Zubereitung bestehend aus einem anti-CD30 Immuntoxine und S,S-ß-Hydroxyectoin bei SCID-Mäusen mit disseminiert wachsenden humanen L540Cy-Tumoren*

### 1. Methoden

In weibliche, 4 Wochen alte SCID Mäuse wurden 1x10⁷, vom Hodgkin-Lymphom abstammende, L540Cy-Zellen i.v. injiziert. Einen Tag später wurden die Tiere mit dem chemisch-gekoppelten anti-CD30 Immuntoxin Ki-4.dgA (50 µg) ohne und mit 1 M S,S-β-Hydroxyectoin i.v. behandelt; parallel dazu wurden Kontrolltiere mit PBS-Puffer ohne und mit1 M S,S-ß-Hydroxyectoin eingesetzt.

Das verwendete Immuntoxin wird derzeit in einer klinischen Phase I-Studie an der Medizinischen Klinik I an austherapierten und rezidivierten Patienten mit Hodgkin-Lymphom eingesetzt. Das unter GMP-Bedingungen verfügbare Immuntoxin wurde mit einer Dosis von 50 µg bei den Mäusen eingesetzt. Als maximal tolerable Dosis wurde mit diesem Immuntoxin in SCID-Mäusen eine Menge von 48 µg dokumentiert.

### 2. Ergebnisse

Die vorläufigen Ergebnisse dieses Versuches sind als Kaplan-Meier-Darstellung der beigefügten Grafik zu entnehmen. Alle Kontrolltiere entwickelten innerhalb von 35 Tagen disseminiert wachsende Tumoren. Diese Tiere wurden getötet und ihr Tumorbefall dokumentiert: makroskopisch sichtbare Tumoren entwickelten sich in den Lymphknoten, den Nieren, den Eierstöcken, dem Thymus, den Kaumuskeln und im Gehirn.

Für die 5 Tiere der PBS-Gruppe ohne und die 2 Tiere der PBS-Gruppe mit S,S-b-Hydroxyectoin wurden nach Kaplan-Meier mittlere Überlebensraten von 33.0 (±0.55) Tagen und 30.0 (±2) Tagen berechnet.

Vier der fünf mit Ki-4.dgA behandelten Tiere starben innerhalb eines Tages an der verabreichten Dosis, das fünfte Tier litt zunächst unter starkem Gewichtsverlust (ca. 3 g) und erholte sich aber innerhalb von 5 Tagen. Im Vergleich zu den Kontrolltieren konnte im Beobachtungszeitraum von bislang 51 Tagen kein Anzeichen einer Tumorentwicklung festgestellt werden.
Alle vier mit Ki-4.dgA + S,S-b-Hydroxyectoin behandelten Tiere sind innerhalb des Beobachtungszeitraumes nicht verstorben und zeigen ebenfalls kein Anzeichen einer Tumorentwicklung.

Der Unterschied zwischen den Gruppen PBS ohne oder mit S,S-ß-Hydroxyectoin und Ki-4.dgA mit S,S-ß-Hydroxyectoin ist statistisch hoch signifikant (P=0.0046) und signifikant (P=0.0177). Ki-4.dgA ohne und mit S,S-ß-Hydroxyectoin unterscheiden sich signifikant (P=0.0237). Außerdem zeichnet sich ein tendenzieller Unterschied zwischen den Gruppen PBS ohne und mit S,S-ß-Hydroxyectoin ab (P=0.0715), der eine schnellere Tumorentwicklung bei S,S-ß-Hydroxyectoin-behandelten Tieren anzeigen könnte.

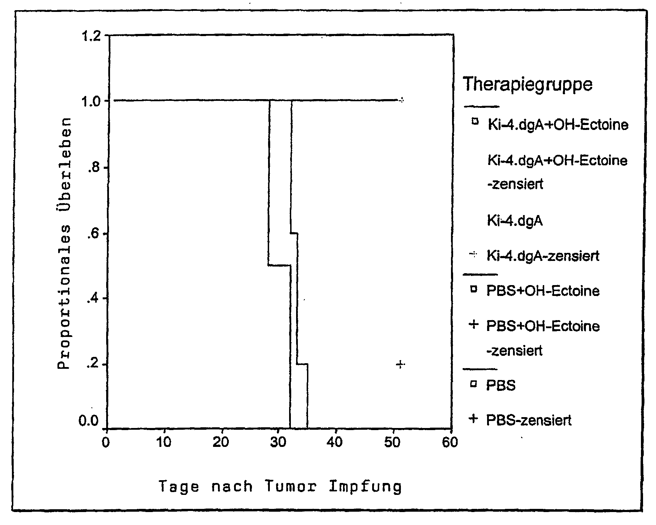

## Patentansprüche

1. Pharmazeutische Zubereitung enthaltend mindestens eine proteinenthaltende Substanz und Ectoin, bevorzugt Hydroxyectoin, wobei die mindestens eine proteinhaltige Substanz ein Antikörper, ein Antikörperfragment, ein rekombinanter mono- oder höhervalenter Antikörper, ein rekombinantes mono- oder höhervalentes Antikörperfragment, ein bispezifischer Antikörper, ein Diabody oder ein daraus sich ableitendes toxisches Konjugat (Immuntoxin) ist.

2. Verwendung der pharmazeutischen Zubereitung nach Anspruch 1 zur Herstellung eines Arzneimittels zur Behandlung von Tumorerkrankungen, Autoimmunerkrankungen, chronischen Entzündungen, Allergien, bakteriellen und viralen Infektionen und dem Vascular Leak Syndrome.

## Claims

1. A pharmaceutical formulation containing at least one protein-containing substance and ectoine, preferably hydroxyectoine, wherein said at least one protein-containing substance is an antibody, an antibody fragment, a recombinant mono- or higher valent antibody, a recombinant mono- or higher valent antibody fragment, a bispecific antibody, a diabody, or a toxic conjugate derived therefrom (immunotoxin).

2. Use of the pharmaceutical formulation according to claim 1 for preparing a medicament for the treatment of tumor diseases, autoimmune diseases, chronic inflammations, allergies, bacterial and viral infections, and the vascular leak syndrome.

## Revendications

1. Préparation pharmaceutique contenant au moins une substance protéinique et de l'ectoine, de préférence de l'hydroxyectoine, dans laquelle la au moins une substance protéinique est un anticorps, un fragment d'anticorps, un anticorps recombinant mono- ou polyvalent, un fragment d'anticorps recombinant mono- ou polyvalent, un anticorps bispécifique, un 'diabody' ou un conjugué toxique (immunotoxine) dérivé d'entre eux.

2. Utilisation de la préparation pharmaceutique selon la revendication 1 pour préparer un médicament destiné au traitement des maladies tumorales, des maladies auto-immunes, des inflammations chroniques, des allergies, des infections bactériennes et virales et du 'Vascular Leak Syndrome'.
